(19) Europäisches Patentamt
European Patent Office
Office européen des brevets

(11) **EP 1 518 495 B1**

(12) **EUROPÄISCHE PATENTSCHRIFT**

(45) Veröffentlichungstag und Bekanntmachung des Hinweises auf die Patenterteilung:
**31.03.2010 Patentblatt 2010/13**

(51) Int Cl.:
**A61B 5/00** *(2006.01)*

(21) Anmeldenummer: **04021094.0**

(22) Anmeldetag: **04.09.2004**

(54) **Verfahren und Gerät zur laufenden Überwachung der Konzentration eines Analyten**

Method and device for continuous monitoring of analyte concentration

Procédé et dispositif de surveillance continue de la concentration d'un analyte

(84) Benannte Vertragsstaaten:
**AT BE BG CH CY CZ DE DK EE ES FI FR GB GR HU IE IT LI LU MC NL PL PT RO SE SI SK TR**

(30) Priorität: **23.09.2003 DE 10343863**

(43) Veröffentlichungstag der Anmeldung:
**30.03.2005 Patentblatt 2005/13**

(73) Patentinhaber:
• **Roche Diagnostics GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AT BE BG CH CY CZ DK EE ES FI FR GB GR HU IE IT LI LU MC NL PL PT RO SE SI SK TR**

(72) Erfinder:
• **Kotulla, Reinhard, Dr.**
**67245 Lambsheim (DE)**
• **Staib, Arnulf, Dr.**
**64380 Rossdorf (DE)**
• **Gillen, Ralph**
**26871 Papenburg (DE)**

(74) Vertreter: **Pfeifer, Hans-Peter et al**
**Durm & Partner**
**Patentanwälte**
**Moltkestrasse 45**
**76133 Karlsruhe (DE)**

(56) Entgegenhaltungen:
**WO-A-02/24065     WO-A-98/42249**
**US-B1- 6 519 705**

• **MYERS K A; TAPLEY B D: "Adaptive sequential estimation with unknown noise statistics" IEEE TRANS AUTOM CONTROL AUG 1976, Bd. AC-21, Nr. 4, August 1976 (1976-08), Seiten 520-523, XP002310881**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung bezieht sich auf ein Verfahren und ein Gerät zur laufenden Überwachung der Konzentration eines Analyten durch Bestimmung von dessen zeitlichem Verlauf im lebenden Körper eines Menschen oder Tieres. Nachfolgend wird hierfür die Bezeichnung "continuous monitoring (CM)" verwendet.

Ein CM-Verfahren und -Gerät ist beispielsweise in

(1) US-Patent 5,507,288

beschrieben. Es geht dabei insbesondere um die kontinuierliche Überwachung der Konzentration von Glucose im Körper eines Patienten. Sie ist von größter medizinischer Bedeutung. Studien haben zu dem Ergebnis geführt, daß äußerst schwerwiegende langfristige Folgen des Diabetes Mellitus (beispielsweise Erblinden aufgrund einer Retinopathie) vermieden werden können, wenn der zeitliche Verlauf der Konzentration der Glucose in vivo kontinuierlich überwacht wird. Dadurch besteht die Möglichkeit, die erforderliche Medikation (Insulin) zu jedem Zeitpunkt exakt zu dosieren und den Blutzuckerspiegel ähnlich wie bei einer gesunden Person ständig innerhalb enger Grenzen zu halten.

Die Erfindung bezieht sich insbesondere auf Glucose-CM. Ergänzend wird auf das Dokument (1) und die darin zitierten weiteren Literaturstellen bezug genommen.

Die Erfindung ist jedoch generell auch für andere Anwendungsfälle geeignet, bei denen der zeitliche Verlauf eines Analyten im lebenden Körper (Nutzsignal) aus einem Meßsignal abgeleitet wird, welches aus zu aufeinanderfolgenden Zeitpunkten gemessenen Meßwerten einer mit der gesuchten Konzentration korrelierenden Meßgröße besteht. Das Meßsignal kann dabei sowohl invasiv als auch nichtinvasiv gemessen werden.

Ein invasives Meßverfahren wird beispielsweise in

(2) US-Patent 6,584,335

beschrieben. Dabei wird eine Hohlnadel mit einer dünnen optischen Faser in die Haut eingestochen, Licht durch die optische Faser unter die Hautoberfläche eingestrahlt und eine Modifikation des Lichts durch Wechselwirkung mit interstitieller Flüssigkeit, die die optische Faser umgibt, gemessen. Das Meßsignal besteht hier aus Meßwerten, die an Licht gewonnen werden, welches nach der Wechselwirkung mittels der optischen Faser in ein Meßgerät zurückgeführt wird. Beispielsweise kann das Meßsignal aus Spektren des Lichts bestehen, die zu aufeinanderfolgenden Zeitpunkten gemessen werden.

Ein anderes Beispiel invasiver Meßverfahren sind Konzentrationsüberwachungen mittels eines in die Haut einstechbaren elektrochemischen Sensors, wobei in diesem Fall eine elektrische Meßgröße, in der Regel ein Strom, als mit der Konzentration des Analyten korrelierende Meßgröße bestimmt wird.

Verschiedene nichtinvasive Verfahren werden in dem Dokument (1) diskutiert. Hierzu gehören spektroskopische Verfahren, bei denen Licht unmittelbar (also ohne Verletzung der Haut) durch die Hautoberfläche in den Körper eingestrahlt und das dabei diffus reflektierte Licht detektiert wird. Derartige Verfahren haben zur Kontrolle des Verlaufs der Sauerstoffsättigung im Blut eine gewisse Bedeutung erlangt. Im Hinblick auf die Analyse von Glucose sind alternative Verfahren bevorzugt, bei denen Licht streng lokalisiert (in der Regel punktförmig) in die Haut eingestrahlt und das Nutzsignal (Verlauf der Glucosekonzentration) aus der räumlichen Verteilung des in der Umgebung der Einstrahlungsstelle aus der Haut austretenden Sekundärlichts gewonnen wird. In diesem Fall wird das Meßsignal also durch das zu aufeinanderfolgenden Zeitpunkten gemessene Intensitätsprofil des Sekundärlichts in der Umgebung der Einstrahlungsstelle gebildet.

Ein gemeinsames Merkmal aller derartigen Verfahren ist, daß die Ermittlung des zeitlichen Verlaufs der Konzentration (Nutzsignal) aus den zu aufeinanderfolgenden Zeitpunkten gemessenen Meßwerten (Meßsignal) mittels eines Mikroprozessorsystems und eines geeigneten Algorithmus erfolgt. Dieser Auswertealgorithmus schließt folgende Teil-Algorithmen ein:

a) einen Filteralgorithmus, durch den Fehler des Nutzsignals reduziert werden, die aus in dem Meßsignal enthaltenen Signalstörungen resultieren und b) einen Umrechnungsalgorithmus, bei dem ein durch Kalibration bestimmter funktionaler Zusammenhang, der die Korrelation zwischen Meßsignal und Nutzsignal beschreibt, verwendet wird.

In der Regel werden diese Teile des Auswertealgorithmus in der genannten Reihenfolge ausgeführt, d.h. aus einem Roh-Meßsignal wird zunächst durch Filterung ein gefiltertes Meßsignal gewonnen und dieses wird in das Nutzsignal umgerechnet. Diese Reihenfolge ist jedoch nicht zwingend. Es kann auch zunächst eine Umrechnung des Roh-Meßsignals in ein Roh-Nutzsignal und dann eine Filterung zu dem endgültigen Nutzsignal erfolgen. Der Auswertealgorithmus kann auch weitere Schritte umfassen, bei denen Zwischengrößen ermittelt werden. Im Rahmen der Erfindung erforderlich ist nur, daß an irgendeiner Stelle des Auswertealgorithmus, die beliebig gewählt werden kann, die beiden genannten Teilalgorithmen a) und b) ablaufen.

Die Erfindung bezieht sich auf Fälle, bei denen Filteralgorithmen in der Zeitdomäne verwendet werden. Gebräuchlich sind insbesondere Kalman-Filteralgorithmen. Nähere Informationen zu derartigen Filteralgorithmen können aus folgenden Literaturzitaten entnommen werden, die teilweise auch chemische und medizinische Anwendungen beschreiben:

(3) S. D. Brown: The Kalman filter in analytical chemistry, Analytica Chimica Acta 181 (1986), 1-26.

(4) K. Gordon: The multi-state Kalman filter in medical monitoring, Computer Methods and Programs in Biomedicine 23 (1986), 147-154.

(5) K. Gordon, A.F.M. Smith: Modelling and monitoring biomedical time series, Journal of the American Statistical Association 85 (1990), 328-337.

(6) US-Patent 5,921,937

(7) EP 0 910 023 A2

(8) WO 01/38948 A2

(9) US-Patent 6,317,662

(10) US-Patent 6,575,905 B2 und WO 02/24065

(11) US-Patent 6,519,705

Wie erwähnt dient der Filteralgorithmus dazu, Störsignale zu entfernen, die in dem Roh-Meßsignal enthalten sind und das Nutzsignal verfälschen würden. Ziel jedes Filteralgorithmus ist es, diese Störungen möglichst vollständig zu eliminieren, gleichzeitig aber eine Verfälschung des Meßsignals zu vermeiden. Dieses Ziel ist bei der in-vivo-Überwachung von Analyten besonders schwer zu erreichen, weil die Meßsignale in der Regel sehr schwach und von starken Störungen überlagert sind. Besondere Probleme werden dadurch verursacht, daß das Meßsignal in der Regel zwei Typen von Störungen enthält, die sich hinsichtlich der Anforderungen an den Filteralgorithmus wesentlich unterscheiden:

-    Meßrauschen: Diese Störsignalanteile folgen einer Normalverteilung mit konstanter Standardabweichung um das richtige (physiologische) Meßsignal

-    nichtphysiologische Signaländerungen, die beispielsweise durch Bewegungen des Patienten und damit verbundene Änderungen der Ankopplung eines Meßsensors an die Haut verursacht werden. Sie sind in der Regel weder um das physiologische Meßsignal normalverteilt, noch ist die Standardabweichung von dem physiologischen Meßsignal konstant. Für solche Störbeiträge des Rohsignals wird nachfolgend die Bezeichnung NNNC (Non Normal Non Constant) - Noise verwendet.

Der Erfindung liegt das technische Problem zugrunde, eine Erhöhung der Genauigkeit von CM-Verfahren durch Verbesserung der Filterung von Störsignalen zu erreichen, insbesondere wenn die erwähnten nicht-physiologischen Signaländerungen auftreten, die weder um das physiologische Meßsignal normalverteilt sind noch eine konstante Standardabweichung von dem physiologischen Meßsignal haben.

Dies wird erfindungsgemäß mittels eines Filteralgorithmus erreicht, der eine Operation einschließt, bei der der Einfluß eines aktuellen Meßwertes auf das Nutzsignal mittels eines Gewichtungsfaktors gewichtet wird ("steuerbarer Filteralgorithmus"), der die Merkmale des Anspruchs 1 aufweist.

Die Erfindung einschließlich bevorzugter Ausführungsformen wird nachfolgend anhand der Figuren näher erläutert. Die darin dargestellten und nachfolgend beschriebenen Besonderheiten können einzeln oder in Kombination verwendet werden, um bevorzugte Ausgestaltungen der Erfindung zu schaffen. Es zeigen:

Fig. 1    ein Blockdiagramm eines erfindungsgemäßen Geräts

Fig. 2    ein Prinzipskizze eines für die Erfindung geeigneten Sensors

Fig. 3    ein Meßsignal eines Sensors gemäß Fig. 2 und

Fig. 4    ein symbolisches Ablaufdiagramm zur Erläuterung des im Rahmen der Erfindung verwendeten Algorithmus

Fig. 5    eine graphische Darstellung typischer Signalverläufe zur Erläuterung des von der Erfindung gelösten Problems

Fig. 6    eine graphische Darstellung von experimentell gewonnenen Meßergebnissen

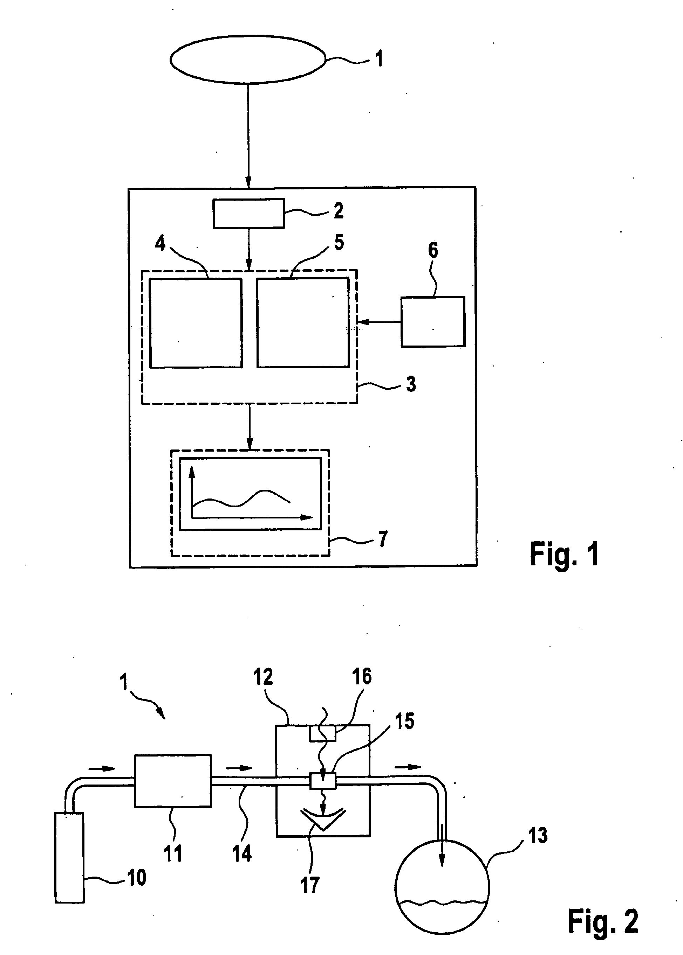

In Fig. 1 sind die wesentlichen Komponenten eines erfindungsgemäßen Geräts dargestellt. Ein Sensor 1 mißt zu aufeinanderfolgenden Zeitpunkten Meßwerte. Dieses Meßsignal wird - im dargestellten Fall drahtlos - an einen Empfänger 2 übertragen, von dem das Meßsignal an eine Auswerteeinheit 3 weitergeleitet wird, die einen Mikroprozessor 4 und einen Datenspeicher 5 enthält. Daten und Befehle können auch über eine Eingabeeinheit 6 an die Auswerteeinheit 3 übermittelt werden. Die Ausgabe von Resultaten erfolgt mittels einer Ausgabeeinheit 7, die ein Display und andere übliche Ausgabemittel einschließen kann. Selbstverständlich erfolgt die Datenverarbeitung in der Auswerteeinheit 3 digital und es sind entsprechende Wandler zur Umwandlung analoger Signale in Digitalsignale vorgesehen. Nähere Erläuterungen hierzu sind nicht erforderlich, weil der grundsätzliche Aufbau derartiger Geräte (beispielsweise aus dem Dokument 1) bekannt ist und die Erfindung für ganz unterschiedliche Meßtechniken geeignet ist, bei denen - wie einleitenden erläutert - unterschiedliche mit dem gewünschten Nutzsignal korrelierende Meßsignale gemessen werden.

Fig. 2 zeigt in Form einer Prinzipskizze einen Sensor 1, bei dem ein implantierbarer Katheter 10 verwendet wird, um aus dem Unterhaut-Fettgewebe interstitielle Flüssigkeit mittels einer Pumpe 11 abzusaugen und durch eine photometrische Meßeinheit 12 in einen Abfallbehälter 13 zu saugen. Die Leitung 14, durch die die interstitielle Flüssigkeit transportiert wird, enthält in der photometrischen Meßeinheit 12 eine transparente Meßzelle 15, in die von einem Lichtsender 16 ausgehendes Primärlicht eingestrahlt wird. Das nach Passieren der Meßzelle 15 resultierende Sekundärlicht wird mittels eines Photodetektors 17 gemessen und mittels einer nicht dargestellten Meßelektronik ein Rohsignal erzeugt, das - wie in Figur 1 beispielhaft dargestellt - an eine Auswerteeinheit 3 weitergeleitet wird.

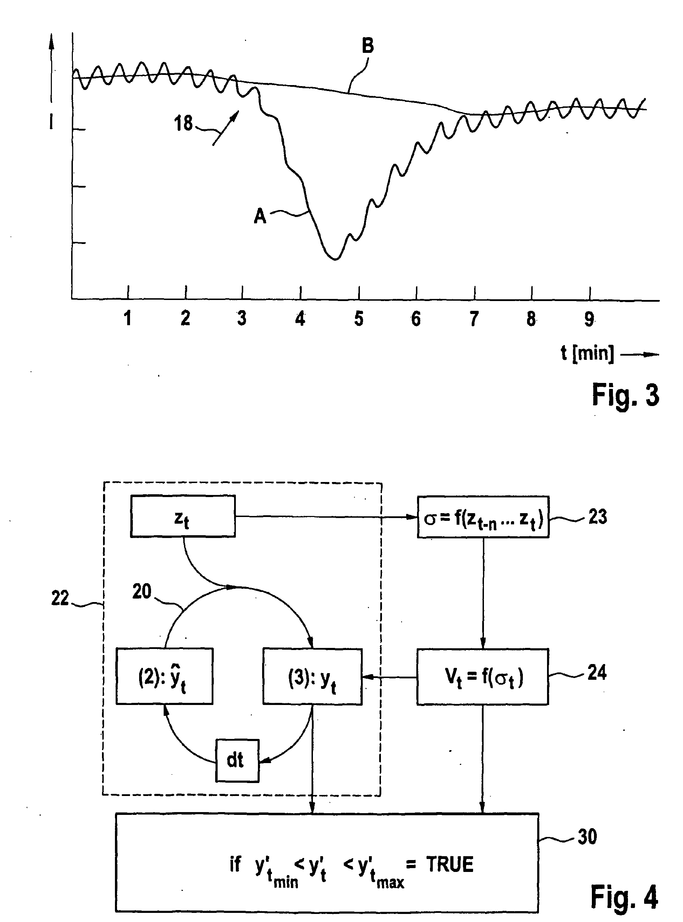

Fig. 3 zeigt als Kurve A den typischen Verlauf eines mit einem Sensor gemäß Figur 2 gewonnenen Roh- Meßsignals, wobei die bei einer bestimmten Wellenlänge gemessene Intensität des Sekundärlichts I gegen die Zeit in Minuten aufgetragen ist. Der Darstellung liegt ein CM-Versuch an einem Probanden zugrunde, wobei das Meßsignal A aus im Abstand von jeweils einer Sekunde gemessenen Meßwerten besteht.

Flußschwankungen des Stroms der interstitiellen Flüssigkeit aus dem Körper in die photometrische Meßeinheit 12 führen zu regelmäßigen relativ kleinen Signalschwankungen, die als "Fluidik-Modulation" bezeichnet werden. Zu dem mit dem Pfeil 18 bezeichneten Zeitpunkt nach etwa drei Minuten tritt eine Hemmung der Flüssigkeitsströmung ein, die beispielsweise durch eine Bewegung des Probanden oder durch das Eindringen eines Zellpartikels in den Katheter 10 verursacht sein kann. Diese Hemmung des Flusses führt zu einem massiven Einbruch des Roh-Meßsignals A. Dies zeigt beispielhaft, daß nicht nur Störsignale auftreten, die als Normalverteilung mit weitgehend konstanter Standardabweichung um das dem tatsächlichen physiologischen Meßwert entsprechende Signal verteilt sind, sondern auch Störbeiträge der hier dargestellten Art, für die diese Bedingung nicht gilt (NNNC-Noise). Ziel der Erfindung ist es, auch in derartigen Fällen die erforderliche Filterung so durchzuführen, daß das in Figur 3 als dünne Linie B gezeichnete Nutzsignal resultiert, welches möglichst genau der tatsächlichen physiologischen Konzentration des Analyten entspricht.

Grundlage eines Filteralgorithmus in der Zeitdomäne, auf den sich die Erfindung bezieht, ist ein Systemmodell, das die zeitliche Entwicklung der interessierenden Variablen und deren Beziehung zueinander beschreibt. Der funktionale Zusammenhang, der die Entwicklung des Systems vom Zeitpunkt t zum Zeitpunkt t+1 beschreibt, lautet:

$$(1) \qquad \mathbf{y}_{t+1} = f_t(\mathbf{y}_t, \mathbf{y}_{t-1}, \ldots, \mathbf{u}_t, \mathbf{u}_{t-1}, \ldots).$$

Darin sind $\mathbf{y}_t$ und $\mathbf{u}_t$ Vektoren, die als Zustandsvektor bzw. als Vektor von Input-Variablen bezeichnet werden. Der Zustandsvektor $\mathbf{y}_t$ enthält die physiologisch interessierenden Variablen sowie eventuell Kontrollvariablen, die eine Kontrolle der Messung ermöglichen, wie weiter unten noch näher erläutert wird. Bei CM-Verfahren gehört hierzu die gesuchte Analytkonzentration, also beispielsweise die Glucose-Konzentration $g_t$ im Blut. Als Kontrollvariable eignet sich die Änderungsgeschwindigkeit der Analytkonzentration $g_t' = dg_t/dt$. Der Zustandsvektor $\mathbf{y}_t$ kann auch meßverfahrens- bezogene Modellvariablen enthalten. Beispielsweise ist es im Falle eines Meßergebnisses der in Figur 3 dargestellten Art vorteilhaft, die Fluidik-Modulation in dem Systemmodell zu berücksichtigen. Diese Modulation läßt sich mittels ihrer zeitlich veränderlichen Frequenz $\omega_t$ und der ebenfalls zeitlich veränderlichen Amplitude $A_t$ beschreiben. Damit ergeben sich für das anhand der Figuren 2 und 3 beschriebene Experiment vier Systemvariable: $g_t, A_t, \omega_t, g_t'$.

In dem Vektor $\mathbf{u}_t$ werden Input-Variablen zusammengefaßt, die in der Regelungstechnik Stellgrößen entsprechen und somit nicht selbst gemessen werden. Im Fall des Glucose-Monitorings ist es beispielsweise zweckmäßig, die gegebene Insulinmenge und zugeführte Broteinheiten als Input-Variablen in diesem Sinne zu berücksichtigen, weil sie die Glucose-Konzentration im Blut beeinflussen. Werden diese Input-Variablen berücksichtigt, hat der Vektor $\mathbf{u}_t$ zwei Elemente: Insulindosis und Broteinheiten. Charakteristisch für die Input-Variablen ist, daß für sie im Rahmen des Filteralgorithmus keine Vorhersage zukünftiger Werte erforderlich ist.

Die genannten Variablen des Zustandsvektors $\mathbf{y}_t$ und des Input-Vektors $\mathbf{u}_t$ sind selbstverständlich nur als Beispiele zu verstehen. Die Erfindung bezieht sich auf sehr unterschiedliche Systeme, die unterschiedliche Systemmodelle erfordern. Ebenso ist es nicht erforderlich, besagte Modelle in diskreter Form zu benutzen: die bekannte alternative kontinuierliche Formulierung unter Verwendung der entsprechenden Differentialgleichungen kann ebenfalls benutzt werden.

Charakteristisch für Filteralgorithmen in der Zeitdomäne, auf die sich die Erfindung bezieht, ist, daß sie eine alternierende Folge von Vorhersagen des Systemzustandes ("Prediktorschritt") und anschließender Korrektur dieser Vorhersage auf Basis eines zusätzlich bestimmten Meßwertes ("Korrektorschritt") einschließen.

In einem Prediktorschritt erfolgt eine Vorhersage des zum Zeitpunkt t aktuellen Wertes der Zustandsvariablen $\mathbf{y}_t$ unter Verwendung der Systemgleichung (1):

$$(2) \qquad \hat{\mathbf{y}}_t = \mathbf{f}_{t-1}(\mathbf{y}_{t-1}, \mathbf{y}_{t-2}, \ldots; \mathbf{u}_{t-1}, \mathbf{u}_{t-2}, \ldots) + \mathbf{w}_{t-1}$$

Darin bezeichnet $\hat{\mathbf{y}}_t$ den mit den Daten des vorherigen Zeitpunktes (t-1) geschätzten (vorhergesagten) Wert des Zustandsvektors zum Zeitpunkt t; $\mathbf{W}_t$ bezeichnet einen Systemfehlervektor.

Im Falle eines rekursiven Filteralgorithmus erfolgt die Berechnung in dem Prediktorschritt nicht jeweils neu unter Berücksichtigung sämtlicher vorhergehender Zeitpunkte (t-1, t-2, t-3...), sondern unter Verwendung einer gewichteten Summe geglätteter Signalwerte. Im Beispiel eines linearen Kalman-Algorithmus kann man die entsprechende Gleichung wie folgt schreiben:

$$(2a) \qquad \hat{\mathbf{y}}_t = \mathbf{A}_{t-1}\mathbf{y}_{t-1} + \mathbf{B}\mathbf{u}_{t-1} + \mathbf{w}_{t-1}$$

Darin ist $\mathbf{A}_t$ die Systemmatrix und $\mathbf{B}$ die Inputmatrix. Im allgemeinen (nichtlinearen) Fall ist $f_t$ vorzugeben oder aus bislang ermittelten Daten zu berechnen.

In dem Korrektorschritt erfolgt eine Korrektur der Vorhersage auf der Grundlage eines aktuellen Meßwertes gemäß

$$(3) \qquad \mathbf{y}_t = \alpha_t \hat{\mathbf{y}}_t + \beta_t \Delta_t$$

Darin ist $\Delta_t$ eine Größe, die ein Maß für die Abweichung eines aktuellen Meßwertes $z_t$ von dem vorhergesagten Wert darstellt und als "Innovation" bezeichnet wird.

$$(4) \qquad \Delta_t = \mathbf{z}_t - \mathbf{h}(\hat{\mathbf{y}}_t)$$

Dabei ist berücksichtigt, daß in der Regel die Systemvariablen nicht unmittelbar beobachtet werden können. Die Verknüpfung zwischen den Meßwerten und den Zustandsvariablen wird über ein Meßmodell (Meßfunktion $\mathbf{h}_t$) hergestellt gemäß:

$$(5) \qquad \mathbf{z}_t = \mathbf{h}_t(\mathbf{y}_t) + \mathbf{v}_t$$

Durch $\mathbf{v}_t$ wird das Rauschen der Meßwerte berücksichtigt.

Für den Fall eines linearen Kalman-Algorithmus (vgl. Gleichung 2a), lautet die Meßgleichung

$$(5a) \qquad \mathbf{z}_t = \mathbf{H}_t \cdot \mathbf{y}_t + \mathbf{V}_t,$$

wobei $\mathbf{H}_t$ die Meßmatrix bezeichnet.

Beispielsweise wird bei der kontinuierlichen Überwachung der Glucose mittels eines elektrochemischen Sensors ein Strom gemessen, der mit der Glucosekonzentration $g_t$ korreliert. In diesem Fall beschreibt $\mathbf{h}_t$ die Korrelation der Zustandsvariablen $g_t$ mit der Meßgröße i (Strom), die ein Element des Vektors $\mathbf{z}_t$ ist.

In dem beschriebenen Beispiel der photometrischen Detektion von Glukose mit filtergestützter Kompensation der Fluidik-Modulation liegt ein nichtlineares Meßmodell vor, das das photometrische Meßsignal $z_t$ mit den Systemvariablen Glukosekonzentration $g_t$, Amplitude $A_t$ sowie Frequenz $\omega_t$ der Fluidikmodulation verknüpft:

$$z_t = g_t + A_t \cdot \sin(\omega_t \cdot t).$$

Gemäß Gleichung (3) wird der Einfluß des aktuellen Meßwertes (in der Innovation $\Delta_t$ enthalten) auf den gefilterten Nutzsignalwert $\mathbf{y}_t$ durch die Faktoren $\alpha_t$ und $\beta_t$ gewichtet, so daß ein steuerbarer Filteralgorithmus vorliegt.

Im Falle eines Kalman-Filters ist $\alpha_t = 1$ für jeden Zeitpunkt und $\beta_t = \mathbf{K}_t$. $\mathbf{K}_t$ bezeichnet den Kalman-Gain. Demzufolge lautet die Korrektorgleichung:

$$(3a) \qquad \mathbf{y}_t = \hat{\mathbf{y}}_t + \mathbf{K}_t \Delta_t$$

Die Bedeutung des Kalman-Gain $\mathbf{K}_t$ und nähere Informationen zu dem Algorithmus können der einschlägigen Literatur, wie weiter oben zitiert, entnommen werden. Anschaulich ausgedrückt bildet der Kalman-Gain ein Maß dafür, wie stark zusätzlich gewonnene Meßwerte berücksichtigt werden. Er wird bei jedem Iterationsschritt des Filteralgorithmus neu berechnet gemäß einer Formel, die sich vereinfachend (für den linearen Fall) wie folgt schreiben läßt:

$$(6) \qquad \mathbf{K}_t = \mathbf{P}_t \cdot \mathbf{H}_t \cdot (\mathbf{P}_t \cdot \mathbf{H}_t + \mathbf{V})^{-1}$$

Dabei bezeichnet $\mathbf{P}_t$ die Kalmanfehlerkovarianzmatrix. $\mathbf{V}$ bezeichnet die Meßfehlerkovarianzmatrix im konventionellen Kalman-Algorithmus.

Gleichung (6) zeigt, daß die Elemente von $\mathbf{K}_t$ Werte größer 0 und kleiner 1 annehmen können. Ist der angenommene Meßfehler $\mathbf{V}$ relativ groß gegenüber der Kalmanfehlerkovarianz $\mathbf{P}_t$, so ist $\mathbf{K}_t$ klein, d.h. der jeweils aktuelle Meßwert wird relativ gering gewichtet. Ist hingegen $\mathbf{V}$ klein gegen $\mathbf{P}_t$ (multipliziert mit $\mathbf{H}_t$), so erfolgt eine starke Korrektur durch den aktuellen Meßwert.

In Figur 4 ist die dem Filtervorgang zugrundeliegende Iterationsschleife 20 graphisch dargestellt. Alternierend erfolgt ein Korrektorschritt, beispielsweise gemäß Gleichung (3) oder (3a), unter Berücksichtigung eines aktuellen Meßwertes $\mathbf{z}_t$ und nach einem Zeitschritt dt ein Prediktorschritt für einen neuen Zeitpunkt, beispielsweise gemäß Gleichung (2) oder (2a). Dieser Teil des Algorithmus wird als Filterkern 22 bezeichnet. Wie erläutert kann er auf unterschiedliche Weise realisiert sein, sofern es sich um einen Algorithmus in der Zeitdomäne handelt und er eine Operation einschließt, bei der der Einfluß eines aktuellen Meßwertes $\bar{\mathbf{z}}_t$ auf das gefilterte Nutzsignal $\mathbf{y}_t$ mittels eines Gewichtungsfaktors $\alpha_t, \beta_t$ bzw. $\mathbf{K}_t$ gewichtet wird.

Ein wesentlicher Beitrag zur Verbesserung der Filterung wird im Rahmen der Erfindung dadurch erreicht, daß auf Basis von in engem zeitlichem Zusammenhang mit der Messung des aktuellen Meßwertes $\mathbf{z}_t$ detektierten Signalschwankungen ein hier mit $\sigma_t$ bezeichneter Signalschwankungsparameter ermittelt wird und die Gewichtung des Einflusses des aktuellen Meßwertes $\mathbf{z}_t$ im Rahmen des Korrektorschrittes in Abhängigkeit von $\sigma_t$ dynamisch adaptiert wird. Dies wird in Figur 4 graphisch verdeutlicht: Das Feld 23 symbolisiert die Ermittlung des Schwankungsparameters $\sigma_t$ als Funktion des Meßsignals in einem vorausgehenden Zeitraum (Meßwerte $z_{t-n}...z_t$). Das Feld 24 symbolisiert die Berechnung des in dem Korrektorschritt berücksichtigten Gewichtungsfaktors (hier beispielhaft die Meßfehlerkovarianz $\mathbf{V}$, die den Kalman-Gain beeinflußt) als Funktion des Signalschwankungsparameters $\sigma_t$. Der Gewichtsfaktor ist eine zeitlich veränderliche (dynamisch adaptierte) Größe (hier $\mathbf{V}_t$).

Die Erfindung zielt nicht darauf ab, unterschiedliche Filtertypen - im Sinne einer Filterbank - durch Anwendung von Gewichtungsfaktoren zu gewichten. Dazu wären nämlich eine Reihe von Systemmodellen analog zu Gleichung (2) zu definieren und zwar für jeden Filter der Filterbank ein Modell. Dies ist in der vorliegenden Erfindung nicht erforderlich und resultiert in geringerer Komplexität des Verfahrens.

Für die in den Schritten 23 und 24 verwendeten funktionalen Zusammenhänge können keine konkreten mathematischen Regeln angegeben werden, weil sie jeweils auf den Einzelfall angepaßt werden müssen. Allgemein gilt jedoch folgendes:

- der Signalschwankungsparameter wird als Funktion von Meßwerten ermittelt, die in engem zeitlichem Zusammenhang mit dem jeweils aktuellen Meßwert stehen. Dadurch wird erreicht, daß die dynamische Adaption des Filters hinreichend rasch erfolgt. Vorzugsweise basiert die Bestimmung des Signalschwankungsparameters auf Meßwerten, die weniger als 30 Minuten, vorzugsweise weniger als 15 Minuten und besonders bevorzugt weniger als 5 Minuten vor der Messung des aktuellen Meßwertes gemessen wurden. Zumindest sollten in dem Algorithmus zur Ermittlung des Signalschwankungsparameters Meßwerte aus den genannten Zeiträumen mitverwendet werden.

- Unabhängig von den im konkreten Fall eingesetzten Formeln gilt das Prinzip, daß bei abnehmender Signalqualität (also beispielsweise Zunahme der Standardabweichung des Meßsignales) der Signalschwankungsparameter und damit der Gewichtungsfaktor (bzw. gegebenenfalls die Gewichtungsfaktoren) in der Richtung verändert werden, daß der Einfluß des jeweils aktuellen Meßwertes vermindert wird.

Als Signalschwankungsparameter eignet sich beispielsweise die Standardabweichung, die wie folgt berechnet werden kann.

Geht man davon aus, daß die Ermittlung der Standardabweichung auf dem aktuellen Meßwert $z$ und vier vorausgehenden Meßwerten $z_1$ bis $z_4$ beruht und bezeichnet man die Differenz zwischen $z$ und den vorausgehenden Werten mit $\delta z$ ($\delta z_n = z - z_n$), so berechnet sich der Mittelwert $\varepsilon$ zu

$$(7) \qquad \varepsilon = \frac{1}{4} \ (\delta z_1 \ + \ \delta z_2 \ + \ \delta z_3 \ + \ \delta z_4)$$

und die Steigung $\varphi$ einer Ausgleichsgeraden zu

$$(8) \qquad \varphi \ = \ \frac{3(\delta z_1 - \delta z_4) + \delta z_2 - \delta z_3}{10}$$

Die Standardabweichung der vier Differenzwerte bezüglich dieser Ausgleichsgeraden ist

$$(9) \qquad \sigma_t \ = \ [ \ \frac{1}{3} \ (\delta z_1 \ - \ (\varepsilon \ + \ 1,5\varphi))^2$$
$$+ \ \frac{1}{3} \ (\delta z_2 \ - \ (\varepsilon \ + \ 0,5\varphi))^2$$
$$+ \ \frac{1}{3} \ (\delta z_3 \ - \ (\varepsilon \ - \ 0,5\varphi))^2$$
$$+ \ \frac{1}{3} \ (\delta z_4 \ - \ (\varepsilon \ - \ 1,5\varphi))^2 \ ] ^{\frac{1}{2}}$$

Auf Basis dieser Standardabweichung läßt sich eine dynamische (zeitabhängige) Meßfehlerkovarianz $V_t$, die in einen Filterkern mit Kalman-Algorithmus eingeht, beispielsweise berechnen gemäß

$$(10) \qquad V_t \ = \ (\sigma_o \ + \ \sigma_t)^\gamma$$

Dabei sind $\sigma_o$ und $\gamma$ konstante, den Filter charakterisierende Parameter, mit deren Einstellung das zeitliche Verhalten des Filters, insbesondere seine Adaptinität, an den jeweiligen Anwendungszweck angepaßt werden kann.

Im Beispiel eines steuerbaren rekursiven Filters hängen die Gewichtungsfaktoren $\alpha_t$, $\beta_t$ aus Gleichung (3) derart vom Signalschwankungsparameter ab, daß mit zunehmendem $\sigma_t$ der Faktor $\alpha_t$ größer und der Faktor $\beta_t$ kleiner wird.

Wie bereits erläutert, stellen die Gleichungen (7) bis (10) nur eine von zahlreichen Möglichkeiten dar, einen Signalschwankungsparameter und auf dessen Basis eine Gewichtungsfaktor für einen steuerbaren Filteralgorithmus in der Zeitdomäne zu ermitteln. Statt der Standardabweichung, die selbstverständlich mit einer abweichenden Anzahl von Meßwerten berechnet werden kann, können auch andere Größen verwendet werden, die ein Maß für die Signalschwankungen in dem einem aktuellen Meßwert vorausgehenden Zeitraum darstellen. Der Begriff "Signalschwankungsparameter" dient allgemein zur Bezeichnung einer mathematischen Größe, die diese Anforderungen erfüllt.

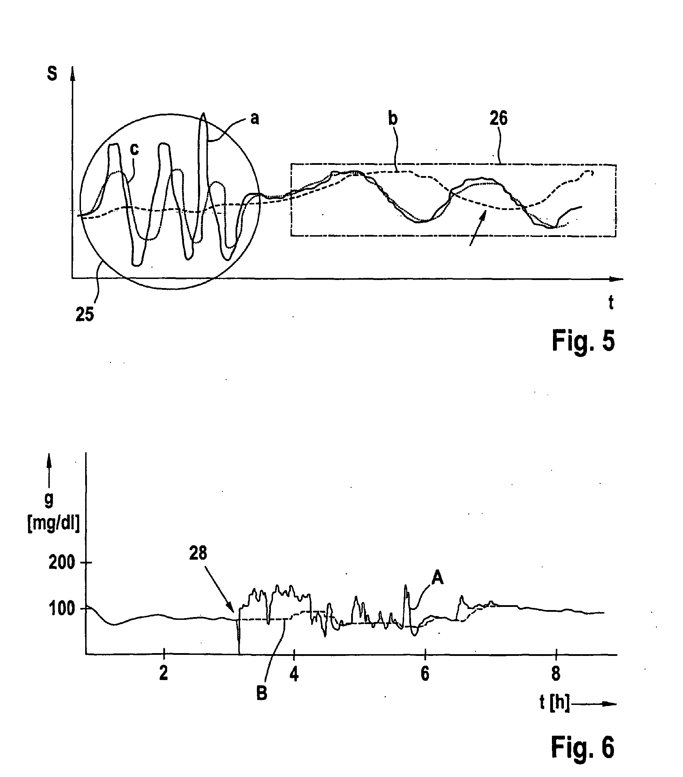

In Figur 5 sind drei typische Verläufe eines Signals S gegen die Zeit t aufgetragen, nämlich:

a) als durchgezogene Linie ein Rohsignal, das in dem mit dem Kreis 25 umrahmten Zeitabschnitt starken nichtphysiologischen Schwankungen unterliegt und in dem mit dem Rechteck 26 umrahmten Zeitabschnitt wesentlich weniger schwankt, wobei diese Schwankungen im wesentlichen physiologisch bedingt sind.
b) in gestrichelter Darstellung ein Nutzsignal, das aus dem Rohsignal a) mittels eines Kalman-Filters gewonnen wurde, dessen Meßfehlerkovarianz entsprechend der Schwankung des Rohsignals in dem Kreis 25 eingestellt war.
c) in gepunkteter Darstellung ein Nutzsignal, das aus dem Rohsignal a) mittels eines Kalman-Filters gewonnen wurde, dessen Meßfehlerkovarianz gemäß dem Verlauf des Rohsignals in dem Rechteck 26 eingestellt war.

Man erkennt, daß bei der Kurve b die starken Schwankungen innerhalb des Kreises 25 gut gefiltert werden, jedoch in dem Rechteck 26 das Signal b den physiologischen Schwankungen des Rohsignals völlig unzureichend folgt. Das Nutzsignal c folgt hingegen den physiologischen Schwankungen in dem Bereich 26 gut während die Filterung der unphysiologischen Schwankungen in dem von dem Bereich 25 unzureichend ist. Der konventionelle Kalman-Filteralgorithmus erlaubt demzufolge keine Einstellung, die bei den dargestellten unterschiedlichen Bedingungen zu einer optimalen Filterung führt. Die Erfindung hingegen erfordert nicht einmal die Kenntnis der maximalen Meßwertschwankungen. Der Filteralgorithmus adaptiert sich selbständig an die Änderungen im Signalverlauf und liefert ein gefiltertes Signal, das in dem Kreis 25 der Kurve b und in dem Rechteck 26 der Kurve c entspricht.

Figur 6 zeigt entsprechende experimentelle Ergebnisse aus einem CM-Versuch zum Glucose-Monitoring an einem Probanden. Dargestellt ist ein bei konventioneller Filterung resultierendes Nutzsignal als durchgezogene Kurve A (Glucosekonzentration in mg/dl) gegen die Zeit in Stunden. Die gestrichelte Kurve B ist das erfindungsgemäß gefilterte Nutzsignal. Zu dem mit dem Pfeil 28 markierten Zeitpunkt beginnt der Proband mit Bewegungen, die den Signalverlauf stören. Die tatsächliche Analytkonzentration ändert sich kaum, aber die durch die Bewegung verursachten Störungen (NNNC-Noise) können von dem konventionellen Filter nicht herausgefiltert werden. Mit der erfindungsgemäßen Filterung erhält man hingegen ein Nutzsignal, das dem physiologischen Glucoseverlauf in sehr guter Näherung folgt.

Eine erhebliche zusätzliche Sicherheit läßt sich erreichen, wenn sich die Filterung nicht nur auf die gesuchte Analytkonzentration, sondern zusätzlich auf mindestens eine weitere Variable erstreckt, die als Kontrollvariable bezeichnet wird. Dies kann eine aus der Analytkonzentration abgeleitete Größe, insbesondere deren erste, zweite oder höhere zeitliche Ableitung sein. Alternativ kommt auch eine zusätzlich gemessene Größe, wie beispielsweise der Fluß der interstitiellen Flüssigkeit bei dem Sensor gemäß Figur 2 in Betracht. Diese Kontrollvariable kann, wie oben (für $g_t'$, $A_t$ und $\omega_t$) erläutert, als Systemvariable in dem Filteralgorithmus mitgeführt werden. Die Filterung erstreckt sich dann auch auf die Kontrollvariable, für die als Ergebnis der Filterung entsprechend zuverlässige geglättete Nutzsignalwerte zur Verfügung stehen. Diese können dann mit Grenzwerten verglichen werden, um beispielsweise Plausibilitätskontrollen durchzuführen. Im Falle der Glucosekonzentration ist zum Beispiel bekannt, daß Änderungsgeschwindigkeiten der Glucosekonzentration von mehr als 3 mg/dl min physiologisch unter gewöhnlichen Bedingungen nicht vorkommen. Wenn der gefilterte Wert der zeitlichen Ableitung $g_t'$ einen höheren Wert annimmt, ist dies ein Zeichen für eine Fehlfunktion. Dies kann durch die in Figur 4 dargestellte Abfrage 30 benutzt werden, bei der der Wert von $y_t'$ mit einem Minimal- und einem Maximalwert verglichen und $y_t$ nur dann als richtig angenommen wird, wenn $y_t'$ innerhalb dieser Grenzen liegt. Ein solcher Vergleich wäre mit dem Nutzsignal A in Figur 6 nicht möglich, weil die unzureichend gefilterten unphysiologischen Schwankungen zu Fehlalarmen führen würden.

## Patentansprüche

1.  Verfahren zur laufenden Überwachung der Konzentration eines Analyten durch Bestimmung von dessen zeitlichem Verlauf im lebenden Körper eines Menschen oder Tieres,
    bei welchem zu aufeinanderfolgenden Zeitpunkten Meßwerte einer mit der gesuchten Konzentration korrelierenden Meßgröße als Meßsignal ($z_t$) gemessen werden und der zeitliche Verlauf der Konzentration als Nutzsignal ($y_t$) aus dem Meßsignal mittels einer Kalibration ermittelt wird,
    wobei
    die Ermittlung des Nutzsignals ($y_t$) aus dem Meßsignal ($z_t$) einen Filteralgorithmus in der Zeitdomäne einschließt, durch den Fehler des Nutzsignals reduziert werden, die aus in dem Meßsignal enthaltenen Störungen resultieren und der Filteralgorithmus eine Operation einschließt, bei der der Einfluß eines aktuellen Meßwertes auf das Nutzsignal mittels eines Gewichtungsfaktors (V) gewichtet wird,

**dadurch gekennzeichnet, daß**

während der laufenden Überwachung auf Basis von in engem zeitlichem Zusammenhang mit der Messung des aktuellen Meßwertes detektierten Signalschwankungen des Messsignals ein zeitlich veränderlicher Signalschwankungsparameter ($\sigma_t$), jeweils für den aktuellen Zeitpunkt, ermittelt wird,

wobei der Signalschwankungsparameter ein Maß für Signalschwankungen des Messsignals in einem dem aktuellen Meßwert vorausgehenden Zeitraum ist und zu dessen Bestimmung Meßwerte verwendet werden, die weniger als 30 Minuten vor der Messung des aktuellen Meßwertes gemessen wurden,

und

der Gewichtungsfaktor dynamisch in Abhängigkeit von dem für den Zeitpunkt der aktuellen Messung ermittelten Signalschwankungsparameters adaptiert wird, wobei der Gewichtungsfaktor in der Richtung verändert wird, dass der Einfluß des jeweils aktuellen Meßwertes bei Zunahme der Standardabweichung der für die Bestimmung des Signalschwankungsparameters verwendeten Meßwerte vermindert wird.

2. Verfahren nach Anspruch 1, **dadurch gekennzeichnet, daß** bei der Bestimmung der Signalschwankungen Meßwerte verwendet werden, die weniger als 15 Minuten, bevorzugt weniger als 5 Minuten vor der Messung des aktuellen Meßwertes gemessen wurden.

3. Verfahren nach einem der vorhergehenden Ansprüche, **dadurch gekennzeichnet, daß** der Filteralgorithmus ein rekursiver Filteralgorithmus ist.

4. Verfahren nach Anspruch 3, **dadurch gekennzeichnet, daß** der Filteralgorithmus ein Kalman-Filteralgorithmus ist.

5. Verfahren nach Anspruch 4, **dadurch gekennzeichnet, daß** der Filteralgorithmus ein linearer Kalman-Filteralgorithmus ist.

6. Verfahren nach einem der vorhergehenden Ansprüche, **dadurch gekennzeichnet, daß** zu den Variablen eines dem Filteralgorithmus zugrundeliegenden Systemmodells eine Kontrollvariable gehört.

7. Verfahren nach Anspruch 6, **dadurch gekennzeichnet, daß** die Kontrollvariable eine zeitliche Ableitung, vorzugsweise die erste zeitliche Ableitung der Analytkonzentration ist.

8. Gerät zur laufenden Überwachung der Konzentration eines Analyten durch Bestimmung von dessen zeitlichem Verlauf im lebenden Körper eines Menschen oder Tieres,

mit einer Meßeinheit (12), durch die zu aufeinanderfolgenden Zeitpunkten Meßwerte einer mit der gesuchten Konzentration korrelierenden Meßgröße als Meßsignal **($z_t$)** gemessen werden und mit einer Auswerteeinheit (3), durch die der zeitliche Verlauf der Konzentration als Nutzsignal **($y_t$)** aus dem Meßsignal mittels einer Kalibration ermittelt wird,

wobei

die Ermittlung des Nutzsignals **($y_t$)** aus dem Meßsignal **($z_t$)** einen Filteralgorithmus in der Zeitdomäne einschließt, durch den Fehler des Nutzsignals reduziert werden, die aus in dem Meßsignal enthaltenen Störungen resultieren und der Filteralgorithmus eine Operation einschließt, bei der der Einfluß eines aktuellen Meßwertes auf das Nutzsignal mittels eines Gewichtungsfaktors (V) gewichtet wird,

**dadurch gekennzeichnet, daß**

während der laufenden Überwachung auf Basis von in engem zeitlichem Zusammenhang mit der Messung des aktuellen Meßwertes detektierten Signalschwankungen des Messsignals ein zeitlich veränderlicher Signalschwankungsparameter ($\sigma_t$), jeweils für den aktuellen Zeitpunkt, ermittelt wird, wobei der Signalschwankungsparameter ein Maß für Signalschwankungen des Messsignals in einem dem aktuellen Meßwert vorausgehenden Zeitraum ist und zu dessen Bestimmung Meßwerte verwendet werden, die weniger als 30 Minuten vor der Messung des aktuellen Meßwertes gemessen wurden,

und

der Gewichtungsfaktor dynamisch in Abhängigkeit von dem für den Zeitpunkt der aktuellen Messung ermittelten Signalschwankungsparameters adaptiert wird, wobei der Gewichtungsfaktor in der Richtung verändert wird, dass der Einfluß des jeweils aktuellen Meßwertes bei Zunahme der Standardabweichung der für die Bestimmung des Signalschwankungsparameters verwendeten Meßwerte vermindert wird.

**Claims**

1. Method for continuous monitoring of the concentration of an analyte by determining its change over time in the living body of a human or animal,

   in which, at sequential points in time, measurement values of a measurement variable correlating with the desired concentration are measured as a measurement signal $(z_t)$ and the change over time of the concentration is determined from the measurement signal as a useful signal $(y_t)$ by means of a calibration,

   wherein

   the determination of the useful signal $(y_t)$ from the measurement signal $(z_t)$ includes a filter algorithm in the time domain, by which errors of the useful signal resulting from noise contained in the measurement signal are reduced, and

   the filter algorithm includes an operation in which the influence of an actual measurement value on the useful signal is weighted by means of a weighting factor (V),

   **characterized in that**,

   during the continuous monitoring, a time dependent signal variation parameter $(\sigma_t)$, related to the actual point in time, is determined on the basis of signal variations of the measurement signal detected in close chronological relation to the measurement of the actual measurement value,

   wherein the signal variation parameter is a measure for signal variations of the measurement signal for a period of time preceding an actual measurement value and is determined on the basis of measurement values including values which were measured less than 30 minutes before the measurement of the actual value; and

   the weighting factor is dynamically adapted as a function of the signal variation parameter determined for the point in time of the actual measurement, the weighting factor being changed in such a direction that the influence of the actual measurement value is reduced with increasing standard deviation of the measurement signal.

2. The method according to Claim 1,

   **characterized in that** measurement values, which were measured less than 15 minutes, and preferably less than 5 minutes before the measurement of the actual measurement value, are used in the determination of the signal variations.

3. The method according to one of the preceding claims,

   **characterized in that** the filter algorithm is a recursive filter algorithm.

4. The method according to Claim 3,

   **characterized in that** the filter algorithm is a Kalman filter algorithm.

5. The method according to Claim 4,

   **characterized in that** the filter algorithm is a linear Kalman filter algorithm.

6. The method according to one of the preceding claims,

   **characterized in that** the variables of a system model upon which the filter algorithm is based comprise a check variable.

7. The method according to Claim 6,

   **characterized in that** the check variable is a time derivative, preferably the first time derivative of the analyte concentration.

8. A device for continuous monitoring of the concentration of an analyte by determining its change over time in the living body of a human or animal,

   comprising a measurement unit (12), by which measurement values of a measurement variable correlating with the desired concentration are measured as the measurement signal $(z_t)$ at sequential points in time, and comprising an analysis unit (3), by which the change over time of the concentration is determined by means of a calibration as a useful signal $(y_t)$ from the measurement signal,

   the determination of the useful signal $(y_t)$ from the measurement signal $(z_t)$ includes a filter algorithm in the time domain, by which errors of the useful signal, which result from noise contained in the measurement signal, are reduced, and

   the filter algorithm includes operations, in which the influence of an actual measurement value on the useful signal is weighted using a weighting factor (V),

   **characterized in that**,

during the continuous monitoring, a time dependent signal variation parameter ($\sigma_t$), related to the actual point in time, is determined on the basis of signal variations of the measurement signal detected in close chronological relation to the measurement of the actual measurement value,

wherein the signal variation parameter is a measure for signal variations of the measurement signal for a period of time preceding an actual measurement value and is determined on the basis of measurement values including values which were measured less than 30 minutes before the measurement of the actual value; and

the weighting factor is dynamically adapted as a function of the signal variation parameter determined for the point in time of the actual measurement, the weighting factor being changed in such a direction that the influence of the actual measurement value is reduced with increasing standard deviation of the measurement signal.

**Revendications**

1. Procédé de surveillance continue de la concentration d'un analyte par détermination de sa variation temporelle dans le corps vivant d'un homme ou d'un animal,

   dans lequel on mesure à des instants successifs des valeurs de mesure d'une grandeur de mesure en corrélation avec la concentration recherchée en tant que signal de mesure ($z_t$) et on détermine à partir dudit signal de mesure, au moyen d'une calibration, la variation temporelle de la concentration en tant que signal utile ($y_t$),

   la détermination du signal utile ($y_t$) à partir du signal de mesure ($z_t$) incluant un algorithme de filtrage dans le domaine temporel, qui permet de réduire des erreurs du signal utile résultant de perturbations contenues dans le signal de mesure, et

   l'algorithme de filtrage incluant une opération consistant à pondérer l'influence d'une valeur de mesure actuelle sur le signal utile au moyen d'un facteur de pondération (V),

   **caractérisé en ce que**

   on détermine au cours de la surveillance continue, sur la base de variations de signal du signal de mesure détectées en rapport temporel étroit avec la mesure de la valeur de mesure actuelle, un paramètre de variation de signal ($\sigma_t$) variable dans le temps pour l'instant actuel,

   ledit paramètre de variation de signal étant une mesure des variations de signal du signal de mesure dans un intervalle de temps précédant la valeur de mesure actuelle et pour la détermination duquel étant utilisées des valeurs de mesure, qui ont été mesurées moins de 30 minutes avant la mesure de la valeur de mesure actuelle, et

   on adapte le facteur de pondération de manière dynamique en fonction du paramètre de variation de signal déterminé pour l'instant de la mesure actuelle, le facteur de pondération étant modifié dans le sens de diminuer l'influence de la valeur de mesure actuelle lorsque l'écart type des valeurs de mesure utilisées pour la détermination du paramètre de variation de signal augmente.

2. Procédé selon la revendication 1, **caractérisé en ce que** l'on utilise lors de la détermination des variations de signal des valeurs de mesure, qui ont été mesurées moins de 15 minutes, de préférence moins de 5 minutes avant la mesure de la valeur de mesure actuelle.

3. Procédé selon l'une des revendications précédentes, **caractérisé en ce que** l'algorithme de filtrage est un algorithme de filtrage récursif.

4. Procédé selon la revendication 3, **caractérisé en ce que** l'algorithme de filtrage est un algorithme de filtrage de Kalman.

5. Procédé selon la revendication 4, **caractérisé en ce que** l'algorithme de filtrage est un algorithme de filtrage linéaire de Kalman.

6. Procédé selon l'une des revendications précédentes, **caractérisé en ce qu'**une variable de contrôle fait partie des variables d'un modèle de système sur lequel est basé l'algorithme de filtrage.

7. Procédé selon la revendication 6, **caractérisé en ce que** la variable de contrôle est une dérivée temporelle, de préférence la première dérivée temporelle de la concentration d'analyte.

8. Appareil de surveillance continue de la concentration d'un analyte par détermination de sa variation temporelle dans le corps vivant d'un homme ou d'un animal,

   avec une unité de mesure (12), permettant de mesurer à des instants successifs des valeurs de mesure d'une grandeur de mesure en corrélation avec la concentration recherchée en tant que signal de mesure ($z_t$), et avec une unité d'évaluation (3), permettant de déterminer à partir dudit signal de mesure, au moyen d'une calibration, la variation temporelle de la concentration en tant que signal utile ($y_t$),

la détermination du signal utile ($y_t$) à partir du signal de mesure ($z_t$) incluant un algorithme de filtrage dans le domaine temporel, qui permet de réduire des erreurs du signal utile résultant de perturbations contenues dans le signal de mesure, et

l'algorithme de filtrage incluant une opération consistant à pondérer l'influence d'une valeur de mesure actuelle sur le signal utile au moyen d'un facteur de pondération (V),

**caractérisé en ce que**

au cours de la surveillance continue, sur la base de variations de signal du signal de mesure détectées en rapport temporel étroit avec la mesure de la valeur de mesure actuelle, un paramètre de variation de signal ($\sigma_t$) variable dans le temps est déterminé pour l'instant actuel,

ledit paramètre de variation de signal étant une mesure des variations de signal du signal de mesure dans un intervalle de temps précédant la valeur de mesure actuelle et pour la détermination duquel étant utilisées des valeurs de mesure, qui ont été mesurées moins de 30 minutes avant la mesure de la valeur de mesure actuelle, et

le facteur de pondération est adapté de manière dynamique en fonction du paramètre de variation de signal déterminé pour l'instant de la mesure actuelle, le facteur de pondération étant modifié dans le sens de diminuer l'influence de la valeur de mesure actuelle lorsque l'écart type des valeurs de mesure utilisées pour la détermination du paramètre de variation de signal augmente.

**Fig. 1**

**Fig. 2**

**Fig. 3**

**Fig. 4**

Fig. 5

Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5507288 A
- US 6584335 B
- US 5921937 B
- EP 0910023 A2
- WO 0138948 A2
- US 6317662 B
- US 6575905 B2
- WO 0224065 A
- US 6519705 B

**In der Beschreibung aufgeführte Nicht-Patentliteratur**

- **S. D. Brown.** *The Kalman filter in analytical chemistry, Analytica Chimica Acta,* 1986, vol. 181, 1-26
- **K. Gordon.** *The multi-state Kalman filter in medical monitoring, Computer Methods and Programs in Biomedicine,* 1986, vol. 23, 147-154
- **K. Gordon ; A.F.M. Smith.** *Modelling and monitoring biomedical time series, Journal of the American Statistical Association,* 1990, vol. 85, 328-337